# EUROPEAN PATENT APPLICATION

(11) **EP 0 672 751 A1**
(43) Date of publication of application: **20.09.1995**
(21) Application number: 93900422.2
(22) Date of filing: 18.12.1992
(51) Int. Cl.: C12N 15/00, C12Q 1/68, C12M 1/00

(54) **GENE MACROMOLECULE REACTION METHOD AND APPARATUS THEREFOR**

(30) Priority: 28.05.1992 JP 137325/92
(71) Applicant: MORINAGA MILK INDUSTRY CO., LTD., Minato-ku, Tokyo-to 108 (JP); ACCESS CO., LTD., Tokyo 101 (JP)
(72) Inventor: KAWASHIMA, Takuji, Kawasaki-shi, Kanagawa 281 (JP); TANAKA, Akiko, Tokyo 155 (JP); SEKI, Reiko, Sagamihara-shi, Kanagawa 229 (JP); ARAKAWA, Ryo, Chiba-shi, Chiba 263 (JP); TANAKA, Hiroaki, Tokyo 155 (JP)
(74) Representative: Holmes, Michael John
(86) International application number: JP9201665
(87) International publication number: WO9324625

(57) **Abstract**

A gene macromolecule reaction method for enzymatically and chemically treating DNA, RNA, their derivatives or their fragments (gene macromolecules) in a solution of high viscosity and/or in the presence of electric field by eliminating thermal fluctuation. An electrostatically oriented-type gene macromolecule reaction device having an alternate current power source, a reaction tank and an electrode submerged in a reaction solution in the reaction tank and connected to the alternate current power source via a lead wire. These method and device make it possible to efficiently and accurately perform enzymatic and chemical synthesis, amplification, cutting, combination, analysis, restoration, duplication, and/or recombination of gene macromolecules.

## Description

### Technical Field

The present invention relates to a method and an equipment for manipulation of genetic macromolecules. More particularly, the present invention relates to a method for efficiently and accurately manipulating DNA, RNA, derivatives thereof or fragments thereof (these are hereinafter referred to as "genetic macromolecules") comprising enzymatic reactions or chemical reactions with excluding thermal fluctuation, and an electrostatically genetic macromolecules orienting-type equipment for manipulation of the genetic macromolecule.

### Background Art

In the recent biotechnology, various methods and various equipments therefor have been developed for synthesis, amplification, cleavage, conjugation, analysis, repair, replcation, recombination, etc. (these operations may be hereinafter referred to as "manipulation") of genetic macromolecules using enzymatic reactions or chemical reactions (such as chemical synthesis, conjugation, modification, analysis, cleavage or decomposition).

A genetic macromolecule has a filamentous molecular structure, and in vivo, maintaining an orderly structure under the effect of biological substances, such as protein and other nucleic acid molecules, thus controlling high-grade information.
In conventional methods and equipments, mimicking and/or modelling bioreactions, such as enzymatic or chemical reactions of these genetic macromolecules, manipulation has been applied to genetic macromolecules dissolved in a solution or these fixed on a solid carrier dispersed in a solution contained in a reaction vessel.

Typical equipments using these techniques include a DNA-RNA synthesizer, a PCR apparatus and a DNA base sequencer [Henry A. Erlich: PCR Technology, Stochton Press, 1989 and Sambrook, et al.: Molecular Cloning: A Laboratory Manual, 2nd ed., Cold Spring Harbor Laboratory Press, 1989].

However, the conventional methods and equipments based on the manipulation of genetic macromolecules in a solution using enzymatic reactions or chemical reactions involve defects of low accuracy and low efficiency in the target manipulation, because the genetic macromolecules in the solution vigorously move under the effect of thermal fluctuation or their three-dimensional structure become low reactive forms by thermal fluctuation. In the case of DNA amplification based on the PCR method, for example, the number of amplifiable DNA bases is limited to about 3.0 Kb.

As a method for manufacturing a filamentous aggregate, techniques have been disclosed, on the other hand, wherein a dielectric solution containing dispersed filaments is arranged between positive and negative electrodes, and the electrostatically oriented filaments are gathered with reserving their orientaion (Japanese Patent Provisional Publication No. 62-161,932, Japanese Patent Provisional Publication No. 62-162,062, Japanese Patent Provisional Publication No. 63-79,924, Japanese Patent Provisional Publication No. 1-208,429, and Japanese Patent Provisional Publication No. 52-33,690).

More recently, Washizu, et al. have reported that application of an alternating current electric field of at least about 1 MHz to DNA causes molecules to be linearly stretched by dielectric polarization to become orienting in parallel with the electric field (SAIBO KOGAKU (Cell Technology), Vol. 8, p. 1011. 1989) and that it is possible to apply a physical manipulation such as fine working to DNA molecules caught by such electrostatic orientation (IEEE transactions on Industry Applications, Vol. 26, No. 6, pp. 1165-1172, 1990).

However, the application of these findings to enzymatic and chemical manipulation of genetic macromolecules, which permits a technology of enzymatic and chemical synthesis, amplification, cleavage, conjugation, analysis, repair, replication and recombination of genetic macromolecules in the presence of alternating current electric field has not get been developed.

### Disclosure of Invention

The present invention was developed to solve the problems as described above, and has an object to provide a method and an equipment for efficiently and accurately carrying out enzymatic or chemical manipulation of genetic macromolecules in a reaction solution with preventing thermal fluctuation thereof.

Tto achieve the above-mentioned object, the present invention provides, a method for manipulating the genetic macromolecules in a reaction solution of high viscosity and/or in the presence of electric field to eliminate thermal fluctuation of the macromolecules, whereof enzymatic or chemical manipulation of genetic macromolecules selected from the group consisting of DNA, RNA, derivatives thereof or fragments thereof.

The present invention provides also an electrostatically genetic macromolecule orienting-type equipment for enzymatic or chemical manipulation of genetic macromolecules selected from the group consisting of DNA, RNA, derivatives thereof or fragments thereof, which comprises an alternating current power source, a reaction vessel, and electrodes submerged in a reaction solution in the reaction vessel and connected to the alternating current power source via lead wires.

In the above-mentioned method and equipment of the present invention, the chemical manipulation is a chemical synthesis, conjugation, modification, analysis, cleavage, decomposition or any combination thereof.

By using the above-mentioned method and equipment of the present invention, it is possible to accurately and efficiently manipulate genetic macromolecules without being affected by characteristics of the base sequence thereof. It is furthermore possible to manipulate genetic macromolecules of a certain base sequence or certain length which has so far been impossible to manipulate by the conventional technology. In addition, the equipment of the present invention can be manufactured at a low cost, and permits accurate and efficient manipulation of genetic macromolecules.

### Brief Description of Drawings

Figs. 1 to 3 are schematic views respectively illustrating examples of configuration of the equipment of the present invention; and
Figs. 4 to 7 show results of agarose electrophoresis for DNA fragments manipulated using the method and the equipment of the present invention.

### Best Mode for Carrying Out the Invention

As a result of various studies carried out on a method for preventing thermal fluctuation of genetic macromolecules in a reaction solution upon manipulating genetic macromolecules, the present inventors found that manipulation of genetic macromolecules in the presence of an electric field, in a reaction solution of which viscosity is adjusted, and in a condition combining those measures were effective as methods capable of certainly preventing thermal fluctuation without allowing adverse effects on an enzymatic or chemical reaction conditions of genetic macromolecules (for example, inactivation of enzyme).

First, the method for adjusting viscosity of the reaction solution comprises the step of adding a substance not affecting manipulation of genetic macromolecules, for example glycerin, sucrose, and polyethylene glycol, to the reaction solution containing genetic macromolecules, thereby adjusting the kinetic viscosity of the reaction solution to high viscosity of about 1.1 to 3.3 cSt (centi-stokes) at 20 °C (hereinafter the kinetic viscosity is the value at 20°C unless otherwise specified). The kinetic viscosity was measured by the method specified in the Japanese Pharmacopoeia ("Japanese Pharmacopoeia Commentary," the 11th revised ed., supervised by the Japan Public Document Association, p. B-277, Hirokawa Shoten, 1986).

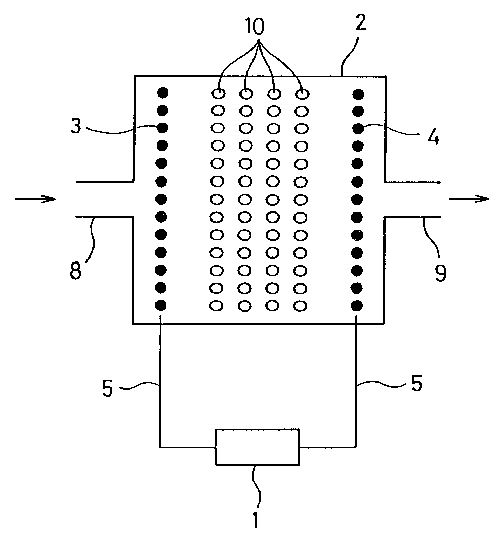

On the other hand, the electric field in the case of manipulating genetic macromolecules in the presence of an electric field is, for example, a high-frequency alternating current electric field of from 1 kHz to 10⁴ kHz and/or strength of from 10⁴ V/m to 10⁷ V/m generated by electrodes coated with an insulating coating having a high dielectric constant, By giving an electric field to the reaction solution, it is possible to prevent folding of genetic macromolecules and increase accuracy of reactions. A high-frequency alternating current electric field can be easily generated by means of a commercially available high-frequency generator. The electrode, of which the surface is coated with a high-dielectric constant insulator is, for example, a platinum electrode presented in Example 1, and a grid-shaped electrode shown in Fig. 3, which is also a preferable form.

Genetic macromolecules can thus be manipulated by the conventional method in a reaction solution having a kinetic viscosity adjusted as above and/or in a reaction solution in the presence of an electric field,

Thus in the followings, Tests to investigate the effect of supressing the thermal fluctuation by the method of the present invention are described.

### Test 1

This test was carried out to investigate the relationship between amplification of DNA fragments and kinetic viscosity of a reaction solution.

### (1) Preparation of sample:

As a DNA sample, λ-phage DNA (purchased from Toyobo Co., Ltd.) was used.

### (2) Procedures:

Using a commercially available PCR reaction reagent kit (purchased from Takara Shuzo Co., Ltd.), glycerin was added to, and uniformly mixed with, a reaction solution to adjust final concentrations of 0, 5, 15 and 30% (by weight; this applies hereafter unless otherwise specified), and sucrose6 to 0, 10, 25 and 30%, respectively; and kinetic viscosity at 20 °C was adjusted to 1, 0.1, 1.5 and 2.3 cSt for glycerin, and 1.0, 1.2, 1.9 and 2.2 cSt for sucrose. With this kit, DNA fragments of 0.5 kb and 2.5 kb on the λ - phage DNA sequence were amplified by the PCR method (25 cycles, each cycle comprising of: 92°C for a minute, 55 °C for two minutes and 72 °C for three minutes), and thus amplified DNA fragments were analyzed by the agarose electrophoresis.

### (3) Results:

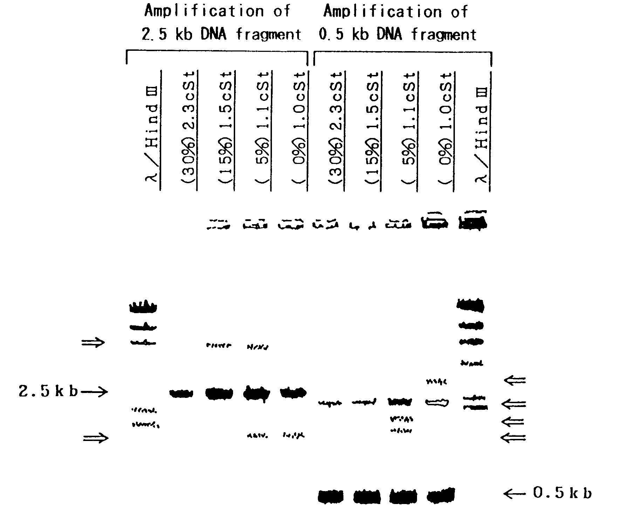

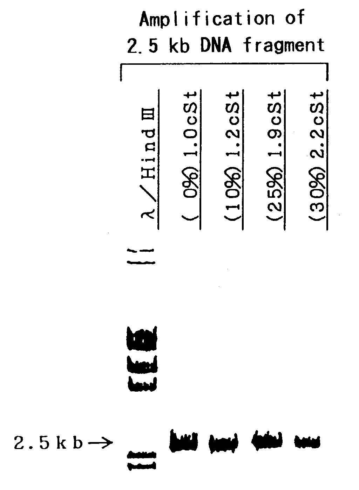

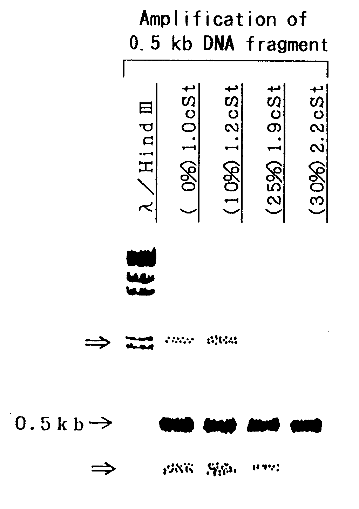

The results of this test are as shown in Figs. 4, 5 and 6. Fig. 4 shows a result of agarose electrophoresis for ampified λ-phage DNA fragments of 0.5 kb and 2.5 kb in the case where glycerin was added, illustrating, from left to right, λ/HindIII (a DNA molecular weight marker, comprising λ-phage DNA cleaved by HindIII restriction enzyme; same applies hereafter), results of amplification of 2.5 kb λ-phage DNA fragment at 2.3, 1.5, 1.1 and 1.0 cSt, followed by those of 0.5 kb DNA fragment at 2.3, 1.5, 1.1 and 1.0 cSt, and λ/HindIII. Fig. 5 shows results of agarose electrophoresis for ampified λ-phage DNA fragments of 2.5 kb in the case where sucrose was added, illustrating, from left to right, λ/HindIII, and the results of the amplification of 2.5 kb λ-phage DNA fragment at 1.0, 1.2, 1.9 and 2.2 cSt. Fig. 6 shows a result of agarose electrophoresis for amplified 0.5 kb λ-phage DNA fragments in the case where sucrose added, illustrating from left to right, λ/HindIII, the results of amplified at 1.0, 1.2, 1.9 and 2.2 cSt.
In Figs. 4 to 6, the mark "→" indicates correctly amplified products, and the mark "⇒" indicates incorrectly amplified products.

In the amplification of the 0.5 kb DNA fragment, as is clear from Fig. 4, the increase in kinetic viscosity of the reaction solution brought about by the addition of glycerin remarkably reduced incorrect amplification of DNA fragments. In the amplification of the 2.5 kb DNA fragment, the increase in kinetic viscosity of the reaction solution reduced incorrect amplification of DNA fragments. And no change was observed in the amount of correctly produced amplified DNA by the increase in kinetic viscosity of the reaction solution. Although not shown in Fig. 4, increase in kinetic viscosity to 3.3 cSt with the addition of glycerin gave a result similar to that for 2.3 cSt.

As is evident from Figs. 5 and 6, the use of sucrose gave no marked change in the amount of production of correctly amplified DNA, as in the case of glycerin, and furthermore, a higher kinetic viscosity corresponded to a higher accuracy. In the method of the present invention, therefore, kinetic viscosity of the reaction solution should be adjusted within a range of from 1.1 to 3.3 cSt, or more preferably, from 1.5 to 2.3 cSt.

Similar results were obtained by adjusting kinetic viscosity of reaction solution even by using other substances (such as polyethylene glycol).

### Test 2

This test was carried out to investigate the relationship between amplification of DNA and the electric field.

### (1) Preparation of sample:

The same sample as in Test 1 was used.

### (2) Procedures:

The PCR method was applied by using a commercially available PCR reagent and Pfu DNA polymerase (purchased from Stratagene Company), according to the manual thereof, or according to the manual except that the time required for changing temperature of the reaction solution from the denaturation temperature to the anneal temperature was reduced to one-third (hereinafter referred to as the "short method"). Provided however that the reaction solution was contained in a plastic bag, which was attached between two electrodes, and was brought into close contact with a DNA thermal cycler heat bath (purchased from Perkin Elmer Setus Company). A high-frequency electric field of from 1 kHz · 2 x 10⁵ V/m to 10⁵ kHz · 2 x 10⁵ V/m was applied between the electrodes. In the above-mentioned reaction equipment a DNA fragment of 2.5 kb on a λ-phage DNA sequence was amplified, and thus amplified DNA fragment was analyzed by the agarose electrophoresis method.

### (3) Results:

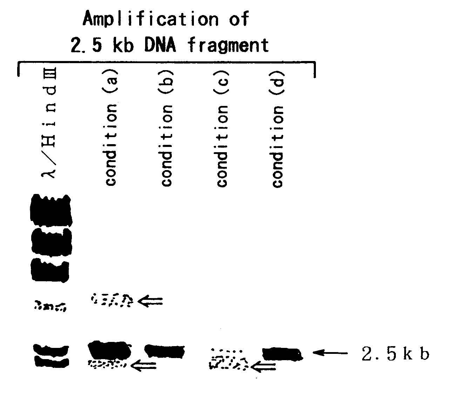

Some of the results of this test are shown in Fig. 7. Fig. 7 illustrates results of agarose electrophoresis for amplification products under (a) an ordinary condition not applying an electric field; (b) a condition comprising application of an electric field of 100 kHz · 2 x 10⁵ V/m; (c) an ordinary condition not applying an electric field by the short method; (d) a condition comprising application of an electric field of 200 kHz · 2 x 10⁵ V/m by the short method. As is clear from Fig. 7, while there is incorrectly amplified DNA fragment marked with "⇒" in addition to the target DNA fragment in (a), correct amplification of the target DNA fragment is observed in (b) and (d), where an electric field is applied.

It was found that application of an electric field as above remarkably reduced incorrect amplification of DNA fragment, with no change in the amount of production of the amplified DNA. Although not shown, application of an electric field of under 1 kHz · 2 x 10⁵ V/m brings about no difference from an ordinary condition not applying an electric field, with increased incorrect amplification of DNA fragments. When applying an electric field of over 10⁵ kHz · 2 x 10⁵ V/m, on the other hand, the enzyme is inactivated by the exothermic reaction. The both cases were thus known to be undesirable.

As is evident from the above-mentioned Tests, it was confirmed that adjustment of viscosity of the reaction solution and presence of an electric field are effective for manipulation of genetic macromolecules, and combination of these methods show a remarkable effect maltiplicatively.

Then, the electrostatically genetic macromolecule orienting-type equipment for manipulating the genetic macromolecule of the present invention is described below with refering the attached drawings.

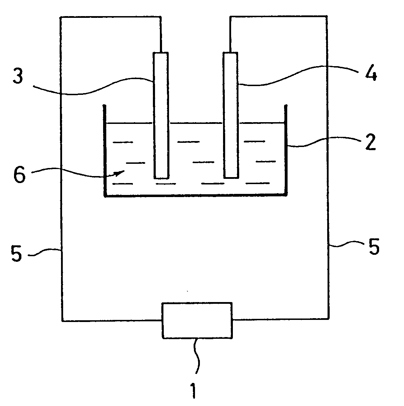

Fig. 1 is a schematic illustration of the basic configuration of the equipment of the present invention.

As shown in Fig. 1, for example, the electrostatically genetic macromolecule orienting-type equipment of the present invention comprises at least an alternating current power source (1), a reaction vessel (2) and electrodes (3) and (4).

The electrodes (3) and (4), comprising platinum electrodes, for example, are respectively connected through lead wires (5) to the alternating current power source (1), and submerged, spaced apart from each other by a distance of 50 to 100 µm, in a reaction solution (6) contained in the reaction vessel (2). As the alternating current power source (1), for example, a combination of an alternating current signal source (FG-350 purchased from Iwatsu Company, or FG163 purchased from NF Kairo Sekkei Block Company), and a high-speed electric power amplifier (4005, 4025, etc. purchased from NF Kairo Sekkei Block Company) is used. And the alternating current power source (1) applies a voltage of, for example, about 50 to 100 Vp-p with 200 kHz to the reaction solution (6). The reaction vessel (2) is made of plastic, for example, an acrylic chamber having a capacity of 500 µl, or a polypropyrene tube having a capacity of 500 µl (purchased from Quality Company). The reaction solution (6) contained in the reaction vessel (2) may be a reaction solution based on the conventional method, or a reaction solution adjusted to a prescribed viscosity as described above, depending upon the subject genetic macromolecule and the manipulation to be applied.

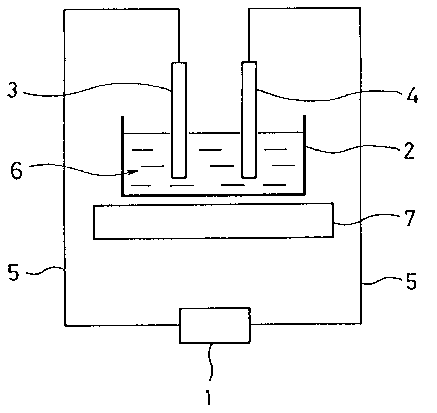

The equipment of the present invention may contain a heat bath (7) arranged under the reaction vessel (2), as shown in the schematic view of Fig. 2. This heat bath (7) is to keep the reaction solution (6) in the reaction vessel (2) at a constant temperature. And, for example, the heat bath (7) may be a DNA thermal cycler (purchased from Perkin Elmer Setus Company).

Fig. 3 is a schematic view illustrating another configuration of the equipment of the present invention.

In the case shown in Fig. 3, a CPG columns (purchased from Applied Biosystems Company) of the DNA Synthesizer (purchased from Applied Biosystems Company) is used as the reaction vessel (2). This improved model of CPG column has a reaction solution inlet (8) and a reaction solution outlet (9), and a 3' terminal base conjugation parts (10) is provided, in addition to the electrodes (3) and (4), in the interior thereof. Glass particles conjugating 3' terminal base or a grid covered with silicon conjugating 3' terminal base may be used as this 3' terminal conjugation parts (10). A distance between the electrodes (3) and (4) can be arbitrarily set at about 2 mm in the case of glass particles, and about 200 µm in the case of a grid. The electrodes (3) and (4) in the CPG column are respectively connected by lead wires (5) to the alternating current power source (1).

Genetic macromolecules can be efficiently and accurately manipulated by using the equipment of the present invention having any of the configurations shown in Figs. 1 to 3, containing a reaction solution based on the conventional method or a reaction solution adjusted to a prescribed viscosity into the reaction vessel, and performing enzymatic reactions or chemical reactions by the application of the conventional method in the presence of an electric field.

The present invention is described below further in detail by means of Examples. It should be noted that the present invention is not limited to the Examples presented hereafter.

### EXAMPLE 1

An equipment as shown in Fig. 2 was tentatively constructed. High frequency electric field was generated by an alternating current power source (1), combinating an alternating current signal source (purchased from Iwatsu Company: FG-350) and a high-speed power amplifier (purchased from NF Kairo Sekkei Block Company: 4005). Electrodes (3) and (4) were commercially available platinum wires having a diameter of 1.0 mm, and were connected by lead wires (5) to the power source. A vessel made of polypropyrene having a capacity of 500 µl (purchased from Quality Company) was used as the reaction vessel (2), and a DNA thermal cycler (purchased from Perkin Elmer Setus Company) was used as the heat bath (7). Because of the usual practice in the PCR method to synthesize a complementary DNA chain by keeping temperature of the reaction solution at about 72°C, the heat bath (7) was provided in this Example.

A trace amount of DNA was amplified by the PCR method using this prototype equipmenrt and a test was carried out by the same method as in the Test 1. As a result, the synthesizing reaction of complementary DNA chain could be conducted efficiently and accurately.

### EXAMPLE 2

An equipment shown in the schematic view of Fig. 3 was tentatively constructed. The same one as in Example 1 was used as the aternating current power source for generating high-frequency electric field. The shape of the CPG column (purchased from Applied Biosystems Company) attached to the DNA synthesizer (purchased from Applied Biosystems Company) was changed, and thus improved CPG column was attached as the reaction vessel (2). Two electrodes (3) and (4) were provided at a distance of about 2 mm in this CPG column and are connected by lead wires (5) to the alternating current power source (1). Glass particles as the 3' terminal base conjugation parts (10) were provided between these electrodes (3) and (4).

DNA synthesis was carried out by the conventional method using this prototype equipment. In the DNA synthesizing reaction in the conventional equipment, folding of synthetic DNA caused by the effect of thermal fluctuation usually causes deceleration or interruption of the synthetic reaction, and generation of various DNA fragments other than the target. In the equipment of the present invention, in contrast, a synthetic reaction of DNA chain could be accomplished efficiently and accurately.

### EXAMPLE 3

Using the equipment used in Example 1 and a commercially available PCR reagent kit (purchased from Takara Shuzo Company), glycerin was added to the reaction solution to reach a final concentration of 30%, and the mixture was uniformly mixed to achieve a kinematic viscosity of 2.3 cSt of the reaction solution.

Using this prototype equipment and the reaction solution, a DNA fragment was amplified by the PCR method, with a 2.5 kb DNA fragment as the template, and the product was analyzed by the same agarose electrophoretic method as in Test 1. As a result, it was confirmed that the target DNA fragment could be amplified accurately and efficiently.

### Industrial Applicability

The method and the equipment of the present invention can be applied widely in various industrial areas where enzymatic or chemical manipulation of genetic macromolecules is utilized, in such basic research and applied areas regarding medicine, food, resources, energy and environment.

## Claims

1. In a method for enzymatic or chemical manipulation of genetic macromolecules selected from the group consisting of DNA, RNA, derivatives thereof and fragments thereof, the improvement comprising the step of manipulating the genetic macromolecules in the presence of electric field to eliminate thermal fluctuation of the macromolecules.

2. The method according to claim 1, wherein said electric field is high-frequency alternating current electric field of from 1 kHz to 10⁴ kHz, or electric field of from 10⁴ V/m to 10⁷ V/m provided by electrodes coated with insulator of high-dielectric constant.

3. The method according to claim 1, wherein the chemical manipulation is chemical synthesis, conjugation, modification, analysis, cleavage, decomposition or any combination thereof.

4. In a method for enzymatic or chemical manipulation of genetic macromolecules selected from the group consisting of DNA, RNA, derivatives thereof and fragments thereof, the improvement comprising the step of manipulating the genetic macromolecules in a reaction solution of a high viscosity to eliminate thermal fluctuation of the macromolecules.

5. The method according to claim 4, wherein the reaction solution has a kinematic viscosity of from 1.1 cSt to 3.3 cSt.

6. The method according to claim 4, wherein the chemical manipulation is chemical synthesis, configuration, modification, analysis, cleavage, decomposition or any combination thereof.

7. In a method for enzymatic or chemical manipulation of genetic macromolecules selected from the group consisting of DNA, RNA, derivatives thereof and fragments thereof, the improvement comprising the step of manipulating the genetic macromolecules in a reaction solution of a high viscosity and in the presence of electric field to eliminate thermal fluctuation of the macromolecules.

8. The method according to claim 7, wherein said reaction solution has a kinematic viscosity of from 1.1 cSt to 3.3 cSt.

9. The method according to claim 7, wherein said electric field is high-frequency alternating current electric field of from 1 kHz to 10⁴ kHz, or electric field of from 10⁴ V/m to 10⁷ V/m provided by electrodes coated with insulator of high-dielectric constant.

10. The method according to claim 7, wherein said chemical manipulation is chemical synthesis, conjugation, modification, analysis, cleavage, decomposition or any combination thereof.

11. An ellectrostatically genetic macromolecule orienting-type equipment for enzymatic or chemical manipulation of genetic macromolecules selected from the group consisting of DNA, RNA, derivatives thereof and fragments thereof, which comprises an alternating current power source, a reaction vessel and electrodes submerged in a reaction solution in the reaction vessel and connected to the alternating current power source via lead wires.
